(19) Europäisches Patentamt

European Patent Office

Office européen des brevets

(11) **EP 1 626 380 A2**

(12) **EUROPÄISCHE PATENTANMELDUNG**

(43) Veröffentlichungstag:
**15.02.2006 Patentblatt 2006/07**

(51) Int Cl.:
*G08B 21/04* *(2006.01)*

(21) Anmeldenummer: **05090215.4**

(22) Anmeldetag: **21.07.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **11.08.2004 DE 102004039635**

(71) Anmelder: **Deutsches Zentrum für Luft- und Raumfahrt e.V.**
**53175 Bonn (DE)**

(72) Erfinder: **Vajen, Hans-Hermann**
**17237 Klein-Vielen (DE)**

(74) Vertreter: **Zucker, Volker**
**Effert, Bressel und Kollegen**
**Patentanwälte**
**Radickestrasse 48**
**12489 Berlin (DE)**

(54) **Verfahren und Vorrichtung zur Abgabe eines Notrufs**

(57) Die Erfindung betrifft ein Verfahren und eine Vorrichtung (1) zur Abgabe eines Notrufs, umfassend eine mobile Kommunikationseinheit (2), eine Positionserfassungseinrichtung und eine Sensorik zum Auslösen eines Notrufs, wobei die Sensorik zum Auslösen eines Notrufs mindestens einen Beschleunigungssensor umfasst, wobei der Notruf mindestens eine Identifikation und eine aktuelle lokale Position umfasst, die Positionserfassungseinrichtung mindestens eine GPS-Einheit oder GPS-Empfänger umfasst, die wiederholt eine Position bestimmt, wobei mittels der Beschleunigungssensoren mindestens die drei translatorischen Beschleunigungen bestimmbar sind, wobei die Vorrichtung (1) Mittel zur Erfassung der rotatorischen Bewegungen umfasst, wobei mittels der Beschleunigungswerte und der rotatorischen Bewegungen zwischen zwei GPS-Messungen eine Bewegungstraktion ermittelbar ist, aus der eine aktuelle lokale Position berechenbar ist, wobei die Bewegungstraktion und/oder die berechnete lokale Position mit dem Notrufsignal übertragbar ist.

Fig. 1

EP 1 626 380 A2

**Beschreibung**

Die Erfindung betrifft ein Verfahren und eine Vorrichtung zur Abgabe eines Notrufs.

Derartige Vorrichtungen sind in jüngster Zeit für die verschiedensten Anwendungsgebiete bekannt geworden. Wesentlich begünstigt werden diese Entwicklungen durch sehr kleine und preisgünstige GPS-Empfänger, die eine verhältnismäßig genaue Lokalisierung ermöglichen. Dieser GPS-Empfänger wird dazu zusammen mit einer GSM-Einheit, die im Falle einer aufgrund der Umgebung bzw. Witterung erfolgenden Abschattung des GPS-Signals die Ortung übernimmt, in einem Mobilfunktelefon integriert, über das im Notfall eine Alarmmeldung mit einer Positionsangabe an eine Zentrale gesendet wird. Dazu wird eine Notrufnummer gespeichert und einer bestimmten Notruftaste zugeordnet. Durch Betätigung dieser Notruftaste wird die Notrufnummer automatisch gewählt und die Positionsangabe an die Zentrale weitergeleitet. Je nach Anwendungsfall wird dann eine Kommunikationsverbindung aufgebaut oder aber die Zentrale leitet Einsatzkommandos wie Polizei oder Feuerwehr zum Ort des Notrufs. Bei einigen Anwendungsfällen, wie beispielsweise Seglern in Seenot, ist die Betätigung der Notruftaste unkritisch, da diese meist körperlich noch wohlauf sind. Bei Personen mit einem hohen Gesundheitsrisiko wie Herzinfarktpatienten oder Schlaganfallpatienten können diese im Notfall derart von einem Anfall überrascht werden, dass diese keine Zeit mehr haben, einen Notruf abzusetzen.

Aus der EP 0 984 414 A2 ist eine Notrufvorrichtung bekannt, umfassend eine mobile Kommunikationseinheit, eine Positionserfassungseinrichtung und einen manuell betätigbaren Alarmschalter, wobei die Notrufvorrichtung mindestens einen Sensor zur Erfassung von Körperfunktionen und einen Speicher mit Referenz-Körperfunktionsdaten umfasst, die in einer Diagnoseeinheit vergleichbar sind, wobei bei Überschreitung vorgebbarer Schwellenwerte ein automatischer Notruf absetzbar ist. Der Sensor ist dabei beispielsweise als Lage-, Temperatur-, Puls-, Blutdruck-, Blutzucker-, Blutsauerstoffkonzentrations- und/oder Schweißsensor ausgebildet. Die Positionserfassungseinrichtung ist als GPS-Empfänger und GSM-Einheit ausgebildet.

Aus der DE 197 29 645 A1 ist ein Personen-Notruf-System bekannt, umfassend wenigstens ein am Körper einer Person zu tragendes Gerät zur drahtlosen Aussendung eines Notrufsignals sowie Empfangsstationen zum Empfangen des jeweiligen, abgesetzten Notrufsignals und zur Übertragung dieses Signals an eine Notrufzentrale, wobei das wenigstens eine Gerät Mittel zum Auslösen eines Notrufs, Mittel zur Bestimmung der aktuellen lokalen Position sowie Mittel zur Speicherung einer die Person identifizierenden Identifikation derart aufweist, dass das jeweilige abgesetzte Notrufsignal die Personen-Identifikation sowie die aktuelle lokale Position des Gerätes enthält. Die Mittel zur Ermittlung der aktuellen lokalen Position des Gerätes werden von einer GPS-Einheit oder einem GPS-Empfänger gebildet. Die Mittel zum Auslösen des Notrufs werden von wenigstens einem geräteinternen Sensor, beispielsweise von einem Neigungssensor und/oder Bewegungssensor und/oder Beschleunigungssensor und/oder Drucksensor gebildet. Nachteilig an den bekannten GPS-Empfängern als Positionsbestimmungsmittel ist, dass diese einen relativ hohen elektrischen Energieverbrauch aufweisen. Dies hat zur Folge, dass bei einer kontinuierlichen Erfassung der Position mittels GPS ein elektrischer Energiespeicher schnell entladen ist. Funkpeilungen sind relativ aufwendig und Messungen im GSM-Netz meist zu ungenau.

Der Erfindung liegt daher das technische Problem zugrunde, eine Vorrichtung und ein Verfahren zur Abgabe eines Notrufs zu schaffen, mittels derer eine kontinuierliche Erfassung einer aktuellen Position bei langer Betriebsdauer möglich ist.

Die Lösung des technischen Problems ergibt sich durch die Gegenstände mit den Merkmalen der Patentansprüche 1 und 10. Weitere vorteilhafte Ausgestaltungen der Erfindung ergeben sich aus den Unteransprüchen.

Hierzu werden mittels der Beschleunigungssensoren mindestens die drei translatorischen Beschleunigungen und mittels weiterer Mittel die rotatorischen Bewegungen erfasst, wobei mittels der Beschleunigungswerte und der rotatorischen Bewegungen (die Winkellage der rotatorischen Freiheitsgrade) zwischen zwei GPS-Messungen eine Bewegungstraktion ermittelt wird, aus der die aktuelle lokale Position berechnet wird, wobei die ermittelte Bewegungstraktion und/oder die berechnete lokale Position mit dem Notrufsignal übertragen wird. Die Berechnung der lokalen Position kann nach der Übertragung der Beschleunigungsdaten auch in einer Zentrale erfolgen. Hierdurch wird erreicht, dass trotz einer nur periodischen GPS-Messung die lokale Position hinreichend genau bestimmt werden kann, wobei die verwendeten Beschleunigungssensoren neben der Ermittlung der Bewegungstraktion auch zum Auslösen des Notrufs verwendet werden können. Dabei sei angemerkt, dass GPS hier stellvertretend für alle geeigneten Satellitennavigationssysteme steht. Ein weiterer Vorteil ist, dass hierdurch sichergestellt ist, dass bei Auslösen des Notrufs auch immer eine aktuelle lokale Position übertragen werden kann. Die bekannten Vorrichtungen gehen davon aus, dass im Notfall stets noch ein GPS-Signal empfangen werden kann, was aber nicht der Realität entspricht, beispielsweise wenn die Person sich in einem stark abschirmenden Gebäude befindet. Bei der Erfindung hingegen steht dann die berechnete Bewegungstraktion von der letzten GPS-Messung ab zur Verfügung, so dass die Lokalisierung auch bei fehlendem GPS-Signal erheblich verbessert werden kann. Ist hingegen eine GPS-Messung möglich, so kann diese selbstverständlich durchgeführt werden und zur Positionsbestimmung alleine oder in Kombination mit der Bewegungstraktion übertragen werden. Erfolgt ohne Auslösung eines Notrufs wieder eine GPS-Messung, so kann zwischenzeitlich die Bewegungstraktion gelöscht werden. Dabei kann die Zeitdauer zwischen zwei GPS-Messungen konstant sein oder aber in Abhängigkeit der erfassten Geschwindigkeit gewählt werden. Wird beispielsweise keine oder nur eine geringe Geschwindigkeit erfasst, so kann die Dauer zwischen zwei GPS-Messungen entsprechend verlängert werden bzw. bei hohen Geschwindigkeiten entsprechend verkürzt werden. Anschaulich lässt sich die Unterscheidung normale Bewegung bzw. Sturz/Fall aus den Werten der Beschleunigung wie folgt beschreiben.

Ist die Vorrichtung konstruktiv so ausgelegt, dass die Erdbeschleunigung (Gravitationskraft) entlang der z-Achse des dreidimensionalen Beschleunigungssensors wirkt, so wird bei ruhender Vorrichtung die lokale Erdbeschleunigung registriert. Ein Sturz kann im einfachsten Fall dadurch erkannt werden, dass der z-Komponente eine kurzzeitige Beschleunigungsspitze überlagert wird und nach kurzer Zeit für beide anderen Komponenten keine Beschleunigung mehr registriert wird. Gleichmäßiges Gehen in einer Ebene wäre durch sehr kleine x-und y-Komponenten der Beschleunigung detektierbar, da bei konstanter Geschwindigkeit die Beschleunigung gleich Null ist. Das Steigen von Treppen würde durch kurzzeitige, sich periodisch mit annähernd gleicher Amplitude zeigende Beschleunigungsereignisse in z-Richtung angezeigt usw.

Dabei ist jedoch zu beachten, dass das Koordinatensystem der Beschleunigungssensoren zum erdbezogenen Koordinatensystem nicht konstant ist, sondern sich nach Lage an der Person verändern kann. Des Weiteren stellt sich das Problem, dass bei einer Drehung der Person um 180° keine nennenswerten Beschleunigungen in x-, y-, und z-Richtung auftreten müssen, sich danach aber die Bewegungsrichtung umkehrt. Wird nun hingegen auch die rotatorische Bewegung erfasst, kann mittels geeigneter Koordinatentransformation das Koordinatensystem der Sensorik auf das inertiale Koordinatensystem der Erde umgerechnet werden. Dabei wird unter rotatorischer Bewegung allgemein verstanden, dass die Winkel der rotatorischen Freiheitsgrade erfasst oder bestimmt werden. Die rotatorische Bewegung wird dabei vorzugsweise auch über eine Bestimmung der rotatorischen Beschleunigungen erfasst. Die konstante Ausrichtung in z-Richtung der Sensorik kann darüber hinaus auch durch eine geeignete mechanische Aufhängung der Sensorik erreicht werden.

Hinsichtlich der konkreten Ausbildung der Beschleunigungssensoren zur Erfassung der translatorischen Beschleunigung sowie der Mittel zur Erfassung der rotatorischen Bewegungen sind verschiedene Ausführungsformen denkbar.

In einer bevorzugten Ausführungsform werden sechs lineare Beschleunigungssensoren verwendet, wobei jeweils drei Beschleunigungssensoren zueinander orthogonal angeordnet sind. Durch diese sechs voneinander unabhängigen Messungen können dann alle sechs Freiheitsgrade bestimmt werden.

Alternativ können zur Bestimmung der translatorischen Beschleunigungen zwei zweidimensionale Beschleunigungssensoren verwendet werden, die ebenfalls orthogonal zueinander angeordnet sind. Dabei ist orthogonal jedoch nicht zwingend, solange die Ebenen sich nur schneiden. Die orthogonale Ausbildung hat jedoch den Vorteil, dass ein kartesisches Koordinatensystem aufgespannt wird, was die Koordinatenbestimmung etwas vereinfacht. Da bei zwei zweidimensionalen Messungen eine redundante Messung erfolgt, kann diese zur Überprüfung der Messergebnisse verwendet werden. Die rotatorischen Bewegungen können ebenfalls über zwei zweidimensionale Beschleunigungssensoren erfasst werden, da dann zusammen mit den Messungen der translatorischen Beschleunigung wieder sechs unabhängige Messungen durchgeführt werden. Alternativ können die rotatorischen Bewegungen auch mittels eines Gyrokreisels erfasst werden.

In einer bevorzugten Ausführungsform sind die Beschleunigungssensoren als piezoresistive Beschleunigungssensoren ausgebildet, da diese sehr kompakt aufgebaut sind und nur einen geringen Stromverbrauch von wenigen mA aufweisen.

In einer weiteren bevorzugten Ausführungsform erfolgt mittels der GPS-Signale eine Inertialisierung des Koordinatensystems der Sensorik und/oder ein Abgleich der Systemzeit. Durch die GPS-Messung erhält man eine hochgenaue Anfangsposition. Weiterhin ist man in der Lage, durch zwei kurz aufeinander folgende Messungen den Vektor der Anfangsgeschwindigkeit zu ermitteln. Dieses sind die Konstanten, die bei der Berechnung des zurückgelegten Weges aus der Momentanbeschleunigung benötigt werden. Die Momentanbeschleunigung wird mit einer definierten Abtastrate ermittelt.

Die Kopplung der Vorrichtung an ein Satellitennavigationssystem hat aber noch weitere Vorteile. Da die Vorrichtung z.B. für die Verfolgung der Bewegung zwischen zwei satellitengestützten Ortsermittlungen genutzt werden kann, können die systematischen Messfehler durch die Satellitenortung verringert werden. Gleichzeitig werden die hochgenauen Zeitinformationen der Navigationssatelliten genutzt, um den internen Zeitmaßstab der informationsverarbeitenden Einheit zu kalibrieren. Damit werden die Fehler bei der Bewegungsverfolgung, die durch fehlerhafte Zeitmessung in der informationsverarbeitenden Einheit verursacht sind, entscheidend verringert. Der zurückgelegte Weg $s_i$ in Richtung i des verwendeten kartesischen Koordinatensystems errechnet sich bei einer gleichförmigen Beschleunigung $a_i$ zu:

$$S_i = 0,5 * a_i + t^2 + v_i * t + i_0 \qquad \{i = x;y;z\}$$

t - Dauer der gleichförmigen Beschleunigung;
$v_i$ - momentane Geschwindigkeit am Anfang des betrachteten Zeitintervalls;
$i_0$ - momentane Koordinate am Anfang des betrachteten Zeitintervalls;

Nach dem Gesetz der Fehlerfortpflanzung ist zu erwarten, dass der systematische Fehler $ds_i$ bei der Ermittlung der Wegstrecke $s_i$ sehr stark vom Fehler bei der Ermittlung der Zeitdauer dt abhängt.

$$ds_i = a_i * t * dt + v_{i0} * dt$$

Die Fehler bei der Ermittlung der anderen Messgrößen gehen lediglich additiv in das Gesamtergebnis ein. Die Möglichkeit des Vergleichs des Zeitnormals der informationsverarbeitenden Einheit mit dem hochgenauen Zeitsignal des Satellitennavigationssystems bietet somit den Vorteil einer genaueren Ortsbestimmung.

Bei der Nutzung der Vorrichtung zur Navigation ist zunächst eine Inertialisierung des internen Koordinatensystems vorzunehmen. Da sich die Lage der Vorrichtung selbst in Bezug auf die verwendeten äußeren Koordinaten ständig ändern kann, ist nach jeder Messung der Bezug zwischen innerem Koordinatensystem, das durch die Anordnung des Sensors in der Vorrichtung vorgegeben ist, und dem äußeren Koordinatensystem, das der Fortführung der Navigation dient, wieder herzustellen. Das geschieht durch eine Koordinatentransformation in der informationsverarbeitenden Einheit oder in der Zentrale. Gegebenenfalls können aus Parametern der Koordinatentransformation ebenfalls Fall- bzw. Sturzereignisse erkannt werden.

Während die satellitengestützte Messung n der Initialisierung der Bewegungsverfolgung mit der beschriebenen Vorrichtung dient, kann die Messung n+1, die nach einer Zeitdauer t vorgenommen wird, einer Korrektur der in der Vorrichtung benutzten Parameter dienen. Mit Hilfe der informationsverarbeitenden Einheit kann daher ein selbstlernender Prozess implementiert werden.

In einer weiteren bevorzugten Ausführungsform kann ein Notruf auch durch Betätigung eines manuellen Alarmschalters ausgelöst werden, da nicht jeder Notsituation ein Sturz oder Fall vorangehen muss. Des Weiteren ist ebenfalls denkbar, mit einer geeigneten Sensorik Körperfunktionen zu erfassen und als Auslösekriterium für einen Notruf zu verwenden, zumindest jedoch deren Daten mit dem Notruf zu versenden.

In einer weiteren bevorzugten Ausführungsform wird bei der Inertialisierung der Vorrichtung die geografische Breite und Höhe erfasst, die daraus resultierende Erdbeschleunigung ermittelt und bei der Kalibration berücksichtigt. Hierdurch kann die Messgenauigkeit entsprechend verbessert werden.

In einer weiteren bevorzugten Ausführungsform wird die Abgabe eines Signals durch die Zentrale angefordert. Die Vorrichtung sendet dann an die Zentrale ein Signal zurück, das eine Identifikation, eine Position und/oder Beschleunigungswerte sowie gegebenenfalls medizinische Daten umfasst. Hierdurch kann eine vorsorgliche Bewegungsverfolgung durch die Zentrale durchgeführt werden, ohne dass ein Auslösekriterium für die Absetzung eines Notrufes erfüllt ist. Dies stellt zum einen eine gewisse Redundanz für die Notrufauslösung dar, zum anderen ermöglicht dies neben einer reinen Notrufeinrichtung andere vorteilhafte Betriebsmöglichkeiten. So kann dies beispielsweise zur permanenten oder bedarfsweise durchgeführten Lokalisierung von Kindern dienen. Dies ist insbesondere deshalb wichtig, da die natürliche Bewegung von Kindern durchaus Beschleunigungswerte erzeugen kann, die bei einem Erwachsenen als Sturz beurteilt werden würden.

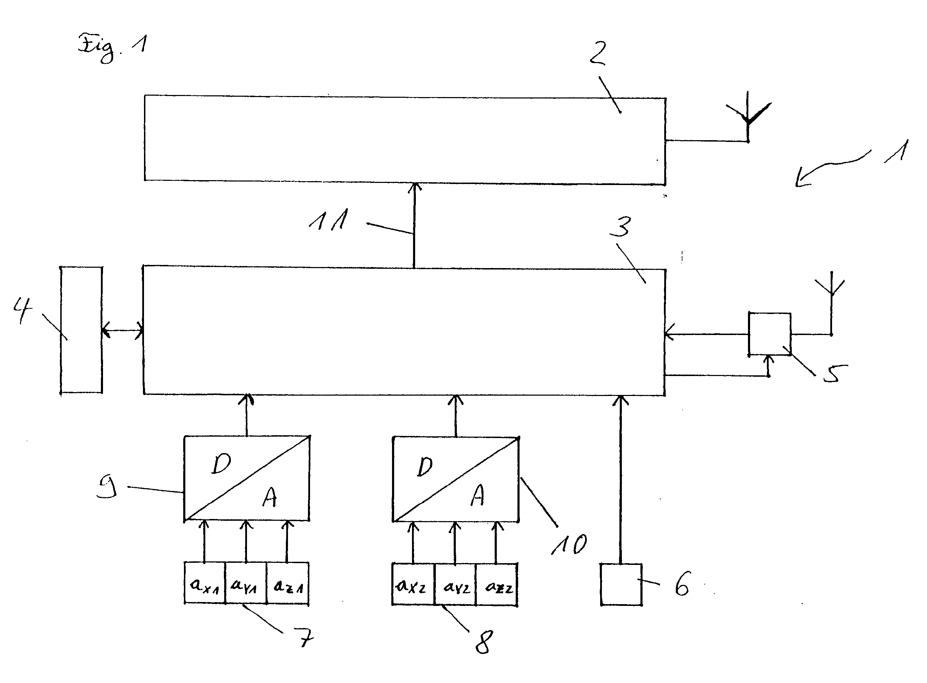

Die Erfindung wird nachfolgend anhand eines bevorzugten Ausführungsbeispieles näher erläutert. Die einzige Figur zeigt ein schematisches Blockschaltbild einer Vorrichtung zur Abgabe eines Notrufs.

Die Vorrichtung 1 zur Abgabe eines Notrufs umfasst eine Kommunikationseinheit 2, einen Mikroprozessor 3 mit zugeordnetem Speicher 4, einen GPS-Empfänger 5, mindestens eine Sensorik 6 zur Erfassung von Körperfunktionen wie beispielsweise Puls, Blutdruck, Blutzuckerwerte etc., zwei weitere Sensoriken 7, 8 zur Erfassung der translatorischen Beschleunigungen $a_x$, $a_y$, $a_z$, die über Analog-/Digital-Wandler 9, 10 mit dem Mikroprozessor 3 verbunden sind. Der Mikroprozessor 3 und die Kommunikationseinheit 2 sind über ein geeignetes Interface 11 miteinander verbunden. Das Interface 11 kann dabei als Luftschnittstelle (z.B. Bluetooth) oder als Steckverbindung ausgebildet sein. In dem Speicher 4 sind Identifikationsdaten abgelegt. Des Weiteren können in dem Speicher 4 Referenzdaten für Körperfunktionsdaten abgelegt sein, die mit den Daten der Sensorik 6 vergleichbar sind. Ebenso können die Daten der Sensorik 6 selbst periodisch im Speicher 4 abgelegt werden und bei Bedarf an eine Zentrale über die Kommunikationseinheit 2 übertragen werden. Mittels der Sensorik 7 und/oder 8 werden signifikante Abweichungen einer Beschleunigung $a_z$ in z-Richtung erfasst und gegebenenfalls nach Vergleich mit Referenzmustern als Sturz oder Fall klassifiziert, wodurch dann ein Notruf abgesetzt wird. Hierzu überträgt dann der Mikroprozessor 3 die relevanten Daten wie Identifikation, Position und/oder Beschleunigungswerte sowie gegebenenfalls medizinische Daten an die Kommunikationseinheit 2, die dann eine Funkverbindung zu einer Zentrale herstellt und diese Daten überträgt.

Die Positionsbestimmung erfolgt dabei nahezu kontinuierlich und soll nun nachfolgend näher erläutert werden. In einem ersten Initialisierungsschritt empfängt der GPS-Empfänger 5 nacheinander zwei GPS-Signale. Aus diesen GPS-Signalen ermittelt der Mikroprozessor 3 einerseits die exakte Position im inertialen Koordinatensystem des GPS sowie die Anfangsgeschwindigkeit V der Vorrichtung durch Bild des Differentials nach der Zeit. Des Weiteren wird die interne Zeit des Mikroprozessors 3 mittels der globalen Zeit vom GPS-Signal abgeglichen. Anhand der Positionsdaten des GPS-Signals kann dann das Koordinatensystem der Sensorik 7, 8 auf das inertiale Koordinatensystem des GPS transformiert werden. Anschließend werden die Beschleunigungswerte $a_{x1}$, $a_{y1}$, $a_{z1}$, $a_{x2}$, $a_{y2}$, $a_{z2}$ der Sensorik 7, 8 erfasst, digitalisiert und dem Mikroprozessor 3 zugeleitet. Anhand der sechs unabhängigen Messungen können neben den translatorischen Beschleunigungen auch die rotatorischen Bewegungen erfasst werden. Somit sind alle Freiheitsgrade der Vorrichtung 1 bekannt bzw. ermittelbar und mittels der Bewegungsgleichung der zurückgelegte Weg berechenbar, da der Anfangsort und die Anfangsgeschwindigkeit aus der GPS-Messung bekannt waren. Hierdurch lässt sich die Position nach der GPS-Messung mittels der Bewegungstraktion aktualisieren. Redundant erfasste Messdaten dienen der Kalibration und/oder Kontrolle. Erfolgt dann wieder eine GPS-Messung, kann die durch Bewegungstraktion ermittelte Position mit der GPS-Position wieder abgeglichen werden. Dabei ist es auch möglich, hier einen selbstlernenden adaptiven Regelvorgang zu implementieren, so dass die Positionsbestimmung durch die Bewegungstraktion sich permanent verbessert.

Wie bereits ausgeführt, wird bei einem Notruf die aktuelle Position mitübertragen. Dies kann dabei in Form einer aus der Bewegungstraktion berechneten Position (Berechnung im Mikroprozessor) oder durch eine Übermittlung der Bewegungstraktion an die Zentrale, die dann die Berechnung vornimmt, erfolgen. Weiter kann vorgesehen sein, dass vor Absetzung des Notrufs überprüft wird, ob ein GPS-Signal empfangbar ist. Ist ein GPS-Signal empfangbar, so kann die daraus ermittelte Position an die Zentrale übertragen werden und ansonsten die durch die Bewegungstraktion ermittelte Position. Es ist jedoch auch möglich, die aus der Bewegungstraktion ermittelte Position stets mit dem Notruf zu übertragen. Da GPS-Messungen einen relativ hohen Energiebedarf haben, kann vorgesehen sein, dass der GPS-Empfänger 5 vom Mikroprozessor 3 gesteuert wird, indem bei geringen Bewegungstraktionen die Zeit zwischen zwei GPS-Messungen verlängert wird und umgekehrt.

**Patentansprüche**

1. Vorrichtung zur Abgabe eines Notrufs, umfassend eine mobile Kommunikationseinheit, eine Positionserfassungseinrichtung und eine Sensorik zum Auslösen eines Notrufs, wobei die Sensorik zum Auslösen eines Notrufs mindestens einen Beschleunigungssensor umfasst, wobei der Notruf mindestens eine Identifikation und eine aktuelle lokale Position umfasst, die Positionserfassungseinrichtung mindestens eine GPS-Einheit oder GPS-Empfänger umfasst, die wiederholt eine Position bestimmt,
**dadurch gekennzeichnet, dass**
mittels der Beschleunigungssensoren mindestens die drei translatorischen Beschleunigungen bestimmbar sind, wobei die Vorrichtung (1) Mittel zur Erfassung der rotatorischen Bewegungen umfasst, wobei mittels der Beschleunigungswerte und der rotatorischen Bewegungen zwischen zwei GPS-Messungen eine Bewegungstraktion ermittelbar ist, aus der eine aktuelle lokale Position berechenbar ist, wobei die Bewegungstraktion und/oder die berechnete lokale Position mit dem Notrufsignal übertragbar ist.

2. Vorrichtung nach Anspruch 1, **dadurch gekennzeichnet, dass** die Vorrichtung mindestens sechs lineare Beschleunigungssensoren umfasst, mittels derer die translatorischen Beschleunigungen und rotatorischen Bewegungen bestimmbar sind.

3. Vorrichtung nach Anspruch 1, **dadurch gekennzeichnet, dass** die Vorrichtung mindestens zwei zweidimensionale Beschleunigungssensoren umfasst, die zueinander orthogonal angeordnet sind, mittels derer die translatorischen Beschleunigungen ermittelbar sind.

4. Vorrichtung nach Anspruch 3, **dadurch gekennzeichnet, dass** die Mittel zur Erfassung der rotatorischen Bewegung als Gyrokreisel oder als zwei zweidimensionale Beschleunigungssensoren ausgebildet sind, die zueinander orthogonal angeordnet sind.

5. Vorrichtung nach einem der vorangegangenen Ansprüche, **dadurch gekennzeichnet, dass** die Beschleunigungssensoren als piezoresistive Beschleunigungssensoren und/oder als Laserkreisel ausgebildet sind.

**6.** Vorrichtung nach einem der vorangegangenen Ansprüche, **dadurch gekennzeichnet, dass** mittels des GPS-Signals eine Inertialisierung des Koordinatensystems der Sensorik und/oder ein Abgleich der Systemzeit erfolgt.

**7.** Vorrichtung nach einem der vorangegangenen Ansprüche, **dadurch gekennzeichnet, dass** die Vorrichtung mindestens einen manuell betätigbaren Alarmschalter umfasst.

**8.** Vorrichtung nach einem der vorangegangenen Ansprüche, **dadurch gekennzeichnet, dass** die Vorrichtung mindestens eine Sensorik (6) zur Erfassung von Körperfunktionen umfasst, deren Daten mit dem Notruf übertragbar sind.

**9.** Vorrichtung nach einem der vorangegangenen Ansprüche, **dadurch gekennzeichnet, dass** bei der Inertialisierung die geografische Breite und Höhe erfasst wird und die daraus resultierende Erdbeschleunigung bei der Kalibration berücksichtigt wird.

**10.** Verfahren zur Abgabe eines Notrufs mittels einer mobilen Kommunikationseinheit (2), einer Positionserfassungseinrichtung und einer Sensorik zum Auslösen eines Notrufs, wobei die Positionserfassungseinrichtung mindestens eine GPS-Einheit oder einen GPS-Empfänger (5) und die Sensorik (7, 8) zum Auslösen eines Notrufs mindestens Beschleunigungssensoren umfasst, mittels derer die drei translatorischen Beschleunigungen bestimmbar sind, sowie Mitteln zum Bestimmen, umfassend folgende Verfahrensschritte.

> a) Inertialisieren des Koordinatensystems der Sensorik (7, 8) mittels einer ersten GPS-Messung, wobei die GPS-Messungen wiederholt werden;
> b) Erfassen der translatorischen Beschleunigungen sowie der rotatorischen Bewegungen der Vorrichtung;
> c) Ermitteln einer Bewegungstraktion anhand der translatorischen Beschleunigungen sowie der rotatorischen Bewegungen;
> d) Vergleichen der Beschleunigungswerte mit Referenzbeschleunigungen, wobei anhand des Vergleichs auf einen Fall und/oder Sturz geschlossen wird und
> e) Auslösen eines Notrufs, wobei der Notruf eine Identifikation und eine aktuelle lokale Position umfasst, die aus der Bewegungstraktion ermittelt wird.

**11.** Verfahren nach Anspruch 10, **dadurch gekennzeichnet, dass** mittels mindestens sechs linearer Beschleunigungssensoren die translatorischen Beschleunigungen und rotatorischen Bewegungen bestimmt werden.

**12.** Verfahren nach Anspruch 10, **dadurch gekennzeichnet, dass** mittels mindestens zweier zweidimensionaler Beschleunigungssensoren, die orthogonal zueinander angeordnet sind, die translatorischen Beschleunigungen ermittelt werden.

**13.** Verfahren nach Anspruch 12, **dadurch gekennzeichnet, dass** mittels zweier zweidimensionaler Beschleunigungssensoren, die orthogonal zueinander angeordnet sind, und/oder eines Gyrokreisels die rotatorischen Bewegungen ermittelt werden.

**14.** Verfahren nach einem der Ansprüche 10 bis 13, **dadurch gekennzeichnet, dass** die Beschleunigungssensoren als piezoresistive Beschleunigungssensoren und/oder als Laserkreisel ausgebildet sind.

**15.** Verfahren nach einem der Ansprüche 10 bis 14, **dadurch gekennzeichnet, dass** mittels der GPS-Signale eine Inertialisierung und/oder ein Abgleich des Koordinatensystems der Sensorik und/oder ein Abgleich der Systemzeit erfolgt.

**16.** Verfahren nach einem der Ansprüche 10 bis 15, **dadurch gekennzeichnet, dass** durch manuelle Betätigung eines Alarmschalters der Notruf abgesetzt wird.

**17.** Verfahren nach einem der Ansprüche 10 bis 16, **dadurch gekennzeichnet, dass** mittels mindestens einer Sensorik Körperfunktionen erfasst werden, deren Daten mit dem Notruf übertragen werden.

**18.** Verfahren nach einem der Ansprüche 10 bis 17, **dadurch gekennzeichnet, dass** bei der Inertialisierung die geografische Breite und Höhe erfasst wird und die daraus resultierende Erdbeschleunigung bei der Kalibration berücksichtigt wird.

**19.** Verfahren nach einem der Ansprüche 10 bis 18, **dadurch gekennzeichnet, dass** bei der Inertialisierung zwei GPS-Signale nacheinander empfangen werden, wobei daraus eine Anfangsgeschwindigkeit bestimmt wird.

**20.** Verfahren nach einem der Ansprüche 10 bis 19, **dadurch gekennzeichnet, dass** von einer Zentrale die Abgabe eines Signals angefordert wird.

Fig. 1

EP 1 626 380 A2